# EUROPEAN PATENT SPECIFICATION

(11) **EP 1 479 691 B1**
(45) Date of publication and mention of the grant of the patent: **15.11.2006**
(21) Application number: 04015696.0
(22) Date of filing: 31.05.2002
(51) Int. Cl.: C07K 14/16, A61K 38/16, A61P 31/18

(54) **Long lasting fusion peptide inhibitors for HIV infection**
Langwirkende Fusionspeptidinhibitoren gegen HIV-Infektion
Inhibiteurs peptidiques de fusion longue durée contre les infections à VIH

(30) Priority: 31.05.2001 US 294241 P
(43) Date of publication of application: 24.11.2004
(62) Divisional of application: 02729737.3
(73) Proprietor: ConjuChem Biotechnologies Inc., Montreal, QC H2X 3Y8 (CA); Erickson, John, Frederick, MD 21703 (US)
(72) Inventor: Erickson, John, Frederick, MD 21703 (US); Bridon, Dominique, P., Ville Mont-Royal, Quebec H3P 2S2 (CA); Robitaille, Martin, Granby, Quebec J2G 6A2 (CA); Krafft, Grant A., Glenview, IL 60025 (US); Xie, Dong, Germantown, MD 20874 (US); Afonina, Elena, Frederick, MD 21702 (US); Liang, Jun, Boyds, MD 20841 (US); De Meyer, Sandra, 2340 Beerse (BE)
(74) Representative: Holmberg, Martin Tor

(56) References cited:
- WO-A-99/59615
- US-A- 6 150 088
- KLIGER Y & SHAI Y: "Inhibition of HIV-1 entry before gp41 folds into its fusion-active conformation" 14 January 2000 (2000-01-14), JOURNAL OF MOLECULAR BIOLOGY, LONDON, GB, PAGE(S) 163-168 , XP004461478 ISSN: 0022-2836 * abstract; table 1 *
- JI H ET AL.: "Buried Polar Interactions and Conformational Stability in the Simian Immunodeficiency Virus (SIV) gp41 Core" BIOCHEMISTRY, vol. 39, 2000, pages 676-685, XP002245319
- NAKAMURA M ET AL: "DESIGN AND SYNTHESIS OF HIGHLY ACTIVE ANTI-HIV PEPTIDE BASED ON GP41-C34 PEPTIDE" 3 October 2001 (2001-10-03), PEPTIDE SCIENCE, PROTEIN RESEARCH FOUNDATION, MINOO,, JP, PAGE(S) 73-76 , XP008018872 ISSN: 1344-7661 * the whole document *

## Description

### FIELD OF INVENTION

This invention relates to C34 peptide derivatives that are inhibitors of viral infection and/or exhibit antifusogenic properties. In particular, this invention relates to C34 derivatives having inhibiting activity against human immunodeficiency virus (HTV), respiratory syncytial virus (RSV), human parainfluenza virus (HPV), measles virus (MeV), and simian immunodeficiency virus (SIV) with long duration of action for the treatment of the respective viral infections.

### BACKGROUND OF THE INVENTION

Membrane fusion events, while commonplace in normal cell biological processes, are also involved in a variety of disease states, including, for example the entry of enveloped viruses into cells. Peptides are known to inhibit or otherwise disrupt membrane fusion-associated events, including, for example, inhibiting retroviral transmission to uninfected cells.

HIV is a member of the lentivirus family of retroviruses, and there are two prevalent types of HIV, HIV-1 and H1V-2, with various strain of each having been identified. HIV targets CD-4+ cells, and viral entry depends on binding of the HIV protein gp120 to the CD4 glycoprotein and a chemokine receptor on cell surface. C34 is known to exhibit anti-viral activity against HIV, including inhibiting CD-4+ cell infection by free virus and/or inhibiting HIV-induced syncytia formation between infected and uninfected CD-4+ cells. The inhibition is believed to occur by binding of C34 to the first heptad repeat region in gp41 and thus preventing the first and second heptad repeat regions from formating the fusigenic hairpin structure.

C34 is known to possess antifusogenic activity, i.e., it has the ability to inhibit or reduce the level of membrane fusion events between two or more entities, e.g., virus-cell or cell-cell, relative to the level of membrane fusion that occurs in the absence of the peptide. More specifically, WO 00/06599 teaches the use of C34 to inactivate gp41, and thus, prevent or reduce HIV-1 entry into cells.

Also WO 99/59615 describes enhancer peptide sequences originally derived from various retroviral envelope (gp41) protein sequences that enhance the pharmacokinetic properties of any core polypeptide to which they are linked. WO 99/59615 further relates to methods for enhancing the pharmacokinetic properties of any core polypeptide through linkage of the enhancer peptide sequences to the core polypeptide.

While many of the anti-viral or anti-fusogenic peptides described in the art exhibit potent anti-viral and/or anti-fusogenic activity, C34, like all such peptides, suffers from short half-life *in vivo,* primarily due to rapid serum clearance and peptidase and protease activity. This in turn greatly reduces its effective anti-viral activity.

There is therefore a need for a method of prolonging the half-life of peptides like C34 *in vivo* without substantially affecting the anti-fusogenic activity.

### SUMMARY OFTHE INVENTION

In accordance with the present invention, there is now provided C34 peptide derivatives having an extended *in vivo* half-life when compared with the corresponding unmodified C34 peptide sequence. More specifically, the present invention is concerned with compounds of Formulae, I and II illustrated below, which are capable of reacting with thiol groups on a blood component, either *in vivo* or *ex vivo,* to form a stable covalent bond.

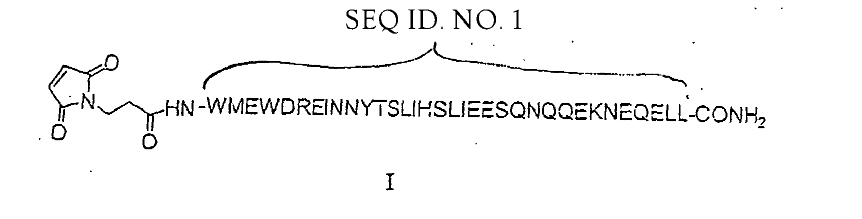

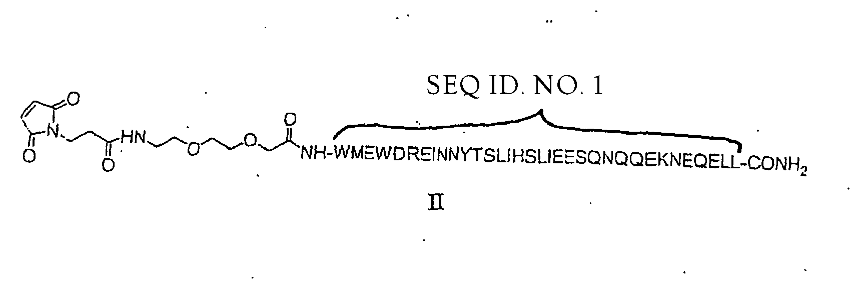

Preferred blood components comprise proteins such as immunoglobulins, including IgG and IgM, serum albumin, ferritin, steroid binding proteins, transferrin, thyroxin binding protein, α-2-macroglobulin etc., serum albumin and IgG being more preferred, and serum albumin being the most preferred.

In another aspect of the invention, there is provided a pharmaceutical composition comprising the derivatives of Formulae I and II in combination with a pharmaceutically acceptable carrier. Such composition is useful for inhibiting the activity of HIV, RSV, HPV, MeV or SIV.

In a further embodiment of the present invention, there is provided a method for inhibiting the activity of HIV, RSV, HPV, MeV or SIV. The method comprises administering to a subject, preferably a mammal, an effective amount of the compounds of Formulae I and II or a conjugate thereof, alone or in combination with a pharmaceutical carrier.

In a further aspect of the present invention, there is provided a conjugate comprising the compounds of Formulae I and II covalently bonded to a blood component

In a further aspect of the present invention, there is provided a method for extending the *in vivo* half-life of the C34 peptide in a subject, the method comprising covalently bonding the compounds of Formulae I and II to a blood component.

### DETAILED DESCRIPTION OF THE INVENTION

The present invention meets these and other needs and is directed to C34 peptides derivatives of Formulae I and II having anti-viral activity and/or anti-fusogenic activity. These C34 peptides derivatives provide for an increased stability *in vivo* and a reduced susceptibility to peptidase or protease degradation. As a result, the compounds of Formulae I and II minimize the need for more frequent, or even continual, administration of the peptides. The present C34 derivatives can be used, e.g., as a prophylactic against and/or treatment for infection of a number of viruses, including human immunodeficiency virus (HIV), human respiratory syncytial virus (RSV), human parainfluenza virus (HPV), measles virus (MeV) and simian immunodeficiency virus (SIV).

The modification made to the native C34 peptide sequence allows it to react with available thiol groups on blood components to form stable covalent bonds. In one embodiment of the invention, the blood component comprises a blood protein, including a mobile blood protein such as albumin, which is most preferred.

The compounds of Formulae I and II inhibit viral infection of cells, by, for example, inhibiting cell-cell fusion or free virus infection. The route of infection may involve membrane fusion, as occurs in the case of enveloped or encapsulated viruses, or some other fusion event involving viral and cellular structures.

The blood components to which the present anti-viral C34 derivatives covalently bonds may be either fixed or mobile. Fixed blood components are non-mobile blood components and include tissues, membrane receptors, interstitial proteins, fibrin proteins, collagens, platelets, endothelial cells, epithelial cells and their associated membrane and membraneous receptors, somatic body cells, skeletal and smooth muscle cells, neuronal components, osteocytes and osteoclasts and all body tissues especially those associated with the circulatory and lymphatic systems. Mobile blood components are blood components that do not have a fixed situs for any extended period of time, generally not exceeding 5 minutes, and more usually one minute. These blood components are not membrane-associated and are present in the blood for extended periods of time in a minimum concentration of at least 0.1 µg/ml. Mobile blood components include serum albumin, transferrin, ferritin and immunoglobulins such as IgM and IgG. The half-life of mobile blood components is at least about 12 hours.

Protective groups may be required during the synthesis process of the present C34 derivative. These protective groups are conventional in the field of peptide synthesis, and can be generally described as chemical moieties capable of protecting the peptide derivative from reacting with other functional groups. Various protective groups are available commercially, and examples thereof can be found in US 5,493,007 which is hereby incorporated by reference. Typical examples of suitable protective groups include acetyl, fluorenylmethyloxycarbonyl (FMOC), t-butyloxycarbonyl (BOC), benzyloxycarbonyl (CBZ), etc. In addition, Table 1 provides both the three letter and one letter abbreviations for amino acids.

**TABLE 1**

| NATURAL AMINO ACIDS AND THEIR ABBREVIATIONS | | |
|---|---|---|
| Name | 3-letter abbreviation | l-letter abbreviation |
| Alanine | Ala | A |
| Arginine | Arg | R |
| Asparagine | Asn | N |
| Aspartic acid | Asp | D |
| Cysteine | Cys | C |
| Glutamic acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The present C34 derivatives may be administered *in vivo* such that conjugation with blood components occurs *in vivo,* or they may be first conjugated to blood components *in vitro* and the resulting conjugated derivative administered *in vivo.*

The present invention takes advantage of the properties of existing anti-viral and antifusogenic peptides. The viruses that may be inhibited by the peptides include, but are not limited to all strains of viruses listed, e.g., in US 6,013,263 and US 6,017,536 at Tables V-VII and IX-XIV therein. These viruses include, e.g., human retroviruses, including HIV-1, HIV-2, and human T-lympocyte viruses (HTLV-I and HTLV-II), and non-human retroviruses, including bovine leukosis virus, feline sarcoma virus, feline leukemia virus, simian immunodeficiency virus (SIV), simian sarcoma virus, simian leukemia, and sheep progress pneumonia virus. Non-retroviral viruses may also be inhibited by the present C34 derivatives, including human respiratory syncytial virus (RSV), canine distemper virus, Newcastle Disease virus, human parainfluenza virus (HPIV), influenza viruses, measles viruses (MeV), Epstein-Barr viruses, hepatitis B viruses, and simian Mason-Pfizer viruses. Non-enveloped viruses may also be inhibited by the present C34 derivatives, and include, but are not limited to, picornaviruses such as polio viruses, hepatitis A virus, enteroviruses, echoviruses, coxsackie viruses, papovaviruses such as papilloma virus, parvoviruses, adenoviruses, and reoviruses.

The focus of the present invention is to modify the C34 peptide sequence to confer to this peptide improved bio-availability, extended half-life and better distribution through selective conjugation of the peptide onto a protein carrier without substantially modifying the peptide's anti-viral properties. The carrier of choice (but not limited to) for this invention would be albumin conjugated through its free thiol.

The present C34 derivatives are designed to specifically react with thiol groups on mobile blood proteins. Such reaction is established by covalent bonding of the peptide modified with a maleimide link to a thiol group on a mobile blood protein such as serum albumin or IgG.

Thiol groups being less abundant *in vivo* than, for example, amino groups, the maleimide-modified C34 peptide of the present invention, will covalently bond to fewer proteins. For example, in albumin (the most abundant blood protein) there is only a single thiol group. Thus, a C34-maleimide-albumin conjugate will tend to comprise approximately a 1:1 molar ratio of C34 peptide to albumin. In addition to albumin, IgG molecules (class II) also have free thiols. Since IgG molecules and serum albumin make up the majority of the soluble protein in blood they also make up the majority of the free thiol groups in blood that are available to covalently bond to the C34 peptide derivative.

Further, even among free thiol-containing blood proteins, including IgGs, specific labeling with a maleimide leads to the preferential formation of a C34-maleimide-albumin conjugate due to the unique characteristics of albumin itself. The single free thiol group of albumin, highly conserved among species, is located at amino acid residue 34 (Cys³⁴). It has been demonstrated recently that the Cys³⁴ of albumin has increased reactivity relative to free thiols on other free thiol-containing proteins. This is due in part to the very low pK value of 5.5 for the Cys³⁴ of albumin. This is much lower than typical pK values for cysteine residues in general, which are typically about 8. Due to this low pK, under normal physiological conditions Cys³⁴ of albumin is predominantly in the ionized form, which dramatically increases its reactivity. In addition to the low pK value of Cys³⁴, another factor which enhances the reactivity of Cys³⁴ is its location, which is in a hydrophobic pocket close to the surface of one loop of region V of albumin. This location makes Cys³⁴ very available to ligands of all kinds, and is an important factor in Cys³⁴'s biological role as free radical trap and free thiol scavenger. These properties make Cys³⁴ highly reactive with maleimide-C34, and the reaction rate acceleration can be as much as 1000-fold relative to rates of reaction of maleimide-C34 with other free-thiol containing proteins.

Another advantage of C34-maleimide-albumin conjugates is the reproducibility associated with the 1:1 loading of C34 to albumin specifically at Cys³⁴. Other techniques, such as glutaraldehyde, DCC, EDC and other chemical activations of, e.g, free amines, lack this selectivity. For example, albumin contains 52 lysine residues, 25-30 of which are located on the surface of albumin and therefore accessible for conjugation. Activating these lysine residues, or alternatively modifying C34 to couple through these lysine residues, results in a heterogenous population of conjugates. Even if 1:1 molar ratios of C34 to albumin are employed, the yield will consist of multiple conjugation products, some containing 0, 1, 2 or more C34 per albumin, and each having C34 randomly coupled at any one or more of the 25-30 available lysine sites. Given the numerous possible combinations, characterization of the exact composition and nature of each conjugate batch becomes difficult, and batch-to-batch reproducibility is all but impossible, making such conjugates less desirable as a therapeutic. Additionally, while it would seem that conjugation through lysine residues of albumin would at least have the advantage of delivering more therapeutic agent per albumin molecule, studies have shown that a 1:1 ratio of therapeutic agent to albumin is preferred. In an article by Stehle, et al., "The Loading Rate Determines Tumor Targeting properties of Methotrexate-Albumin Conjugates in Rats," Anti-Cancer Drugs, VoL 8, pp. 677-685 (1988), incorporated herein in its entirety, the authors report that a 1:1 ratio of the anti-cancer methotrexate to albumin conjugated via glutaraldehyde gave the most promising results. These conjugates were preferentially taken up by tumor cells, whereas conjugates bearing 5:1 to 20:1 methotrexate molecules had altered HPLC profiles and were quickly taken up by the liver *in vivo*. It is postulated that at these higher ratios, conformational changes to albumin diminish its effectiveness as a therapeutic carrier.

Through controlled administration of the present C34 derivatives *in vivo,* one can control the specific labeling of albumin and IgG *in vivo.* In typical administrations, 80-90% of the administered C34 derivatives will label albumin and less than 5% will label IgG. Trace labeling of free thiols such as glutathione will also occur. Such specific labeling is preferred for *in vivo* use as it permits an accurate calculation of the estimated half-life of C34.

In addition to providing controlled specific *in vivo* labeling, the present C34 derivatives can provide specific labeling of serum albumin and IgG *ex vivo.* Such *ex vivo* labeling involves the addition of the C34 derivatives to blood, serum or saline solution containing serum albumin and/or IgG. Once conjugation has occurred *ex vivo* with the C34 derivative, the blood, serum or saline solution can be readministered to the patient's blood for *in vivo* treatment, or lyophilized.

The present C34 derivatives may be synthesized by standard methods of solid phase peptide chemistry well known to any one of ordinary skill in the art. For example, the peptide may be synthesized by solid phase chemistry techniques following the procedures described by Steward et al. in *Solid Phase Peptide Synthesis,* 2nd Ed., Pierce Chemical Company, Rockford, III., (1984) using a Rainin PTI Symphony synthesizer. Similarly, peptides fragments may be synthesized and subsequently combined or linked together to form the C34 peptide sequence (segment condensation).

For solid phase peptide synthesis, a summary of the many techniques may be found in Stewart et al. in *"Solid Phase Peptide Synthesis",* W. H. Freeman Co. (San Francisco), 1963 and Meienhofer, *Hormonal Proteins and Peptides,* 1973, 2 46. For classical solution synthesis, see for example Schroder et al. in *"The Peptides",* volume 1, Acacemic Press (New York). In general, such method comprise the sequential addition of one or more amino acids or suitably protected amino acids to a growing peptide chain on a polymer. Normally, either the amino or carboxyl group of the first amino acid is protected by a suitable protecting group. The protected and/or derivatized amino acid is then either attached to an inert solid support or utilized in solution by adding the next amino acid in the sequence having the complimentary (amino or carboxyl) group suitably protected and under conditions suitable for forming the amide linkage. The protecting group is then removed from this newly added amino acid residue and the next amino acid (suitably protected) is added, and so forth.

After all the desired amino acids have been linked in the proper sequence, any remaining protecting groups (and any solid support) are cleaved sequentially or concurrently to afford the final peptide. By simple modification of this general procedure, it is possible to add more than one amino acid at a time to a growing chain, for example, by coupling (under conditions which do not racemize chiral centers) a protected tripeptide with a properly protected dipeptide to form, after deprotection, a pentapeptide.

A particularly preferred method of preparing the present C34 derivatives involves solid phase peptide synthesis wherein the amino acid α-N-terminal is protected by an acid or base sensitive group. Such protecting groups should have the properties of being stable to the conditions of peptide linkage formation while being readily removable without destruction of the growing peptide chain or racemization of any of the chiral centers contained therein. Examples of N-protecting groups and carboxy-protecting groups are disclosed in Greene, "Protective Groups In Organic Synthesis," (John Wiley & Sons, New York pp. 152-186 (1981)), which is hereby incorporated by reference. Examples of N-protecting groups comprise, without limitation, loweralkanoyl groups such as formyl, acetyl ("Ac"), propionyl, pivaloyl, t-butylacetyl and the like; other acyl groups include 2-chloroacetyl, 2-bromoacetyl, trifluoroacetyl, trichloroacetyl, phthalyl, o-nitrophenoxy-acetyl, -chlorobutyryl, benzoyl, 4-chlorobenzoyl, 4-bromobenzoyl, 4-nitrobenzoyl and the like; sulfonyl groups such as benzenesulfonyl, p-toluenesulfonyl, o-nitrophenylsulfonyl, 2,2,5,7,8-pentamethylchroman-6-sulfonyl (pmc), and the like; carbamate forming groups such as t-amyloxycarbonyl, benzyloxycarbonyl, p-chlorobenzyloxycarbonyl, p-methoxy-benzyloxycarbonyl, p-nitrobenzyloxycarbonyl, 2-nitrobenzyloxycarbonyl, p-bromo-benzyloxycarbonyl, 3,4-dimethoxybenzyloxycarbonyl, 3,5-dimethoxybenzyloxycarbonyl, 2,4-dimethoxybenzyloxycarbonyl, 4-ethoxybenzyloxycarbonyl, 2-nitro-4,5-dimethoxy-benzyloxycarbonyl, 3,4,5-trimethoxybenzyloxycarbonyl, 1-(p-biphenylyl)-1-methyl-ethoxycarbonyl, α,α-dimethyl-3,5-dimethoxybenzyloxycarbonyl, benzhydryloxycarbonyl, t-butyloxycarbonyl (boc), diisopropylmethoxycarbonyl, isopropyloxycarbonyl, ethoxy-carbonyl, methoxycarbonyl, allyloxycarbonyl, 2,2,2,-trichloroethoxycarbonyl, phenoxy-carbonyl, 4-nitrophenoxycarbonyl, fluorenyl-9-methoxycarbonyl, isobornyloxycarbonyl, cyclopentyloxycarbonyl, adamantyloxycarbonyl, cyclohexyloxycarbonyl, phenylthiocarbonyl and the like; arylalkyl groups such as benzyl, biphenylisopropyloxycarbonyl, triphenylmethyl, benzyloxymethyl, 9-fluorenylmethyloxycarbonyl (Fmoc) and the like and silyl groups such as trimethylsilyl and the like. Preferred α-N-protecting group are o-nitrophenylsulfenyl; 9-fluorenylmethyl oxycarbonyl; t-butyloxycarbonyl (boc), isobornyloxycarbonyl; 3,5-dimethoxybenzyloxycarbonyl; t-amyloxycarbonyl; 2-cyano-t-butyloxycarbonyl, and the like, 9-fluorenyl-methyloxycarbonyl (Fmoc) being more preferred, while preferred side chain N-protecting groups comprise 2,2,5,7,8-pentamethylchroman-6-sulfonyl (pmc), nitro, p-toluenesulfonyl, 4-methoxybenzene-sulfonyl, Cbz, Boc, and adamantyloxycarbonyl for side chain amino groups like lysine and arginine; benzyl, o-bromobenzyloxycarbonyl, 2,6-dichlorobenzyl, isopropyl, t-butyl (t-Bu), cyclohexyl, cyclopenyl and acetyl (Ac) for tyrosine; t-butyl, benzyl and tetrahydropyranyl for serine; trityl, benzyl, Cbz, p-toluenesulfonyl and 2,4-dinitrophenyl for histidine; formyl for tryptophan; benzyl and t-butyl for aspartic acid and glutamic acid; and triphenylmethyl (trityl) for cysteine.

A carboxy-protecting group conventionally refers to a carboxylic acid protecting ester or amide group. Such carboxy protecting groups are well known to those skilled in the art, having been extensively used in the protection of carboxyl groups in the penicillin and cephalosporin fields as described in US-3,840,556 and US-3,719,667, the disclosures of which are hereby incorporated herein by reference. Representative carboxy protecting groups comprise, without limitation, C₁-C₈ loweralkyl; arylalkyl such as phenethyl or benzyl and substituted derivatives thereof such as alkoxybenzyl or nitrobenzyl groups; arylalkenyl such as phenylethenyl; aryl and substituted derivatives thereof such as 5-indanyl; dialkylaminoalkyl such as dimethylaminoethyl; alkanoyloxyalkyl groups such as acetoxymethyl, butyryloxymethyl, valeryloxymethyl, isobutyryloxymethyl, isovaleryloxymethyl, 1-(propionyloxy)-1-ethyl, 1-(pivaloyloxyl)-1-ethyl, 1-methyl-1-(propionyloxy)-1-ethyl, pivaloyloxymethyl, propionyloxymethyl; cycloalkanoyloxyalkyl groups such as cyclopropylcarbonyloxymethyl, cyclobutylcarbonyloxymethyl, cyclo-pentylcarbonyloxymethyl, cyclohexylcarbonyloxymethyl; aroyloxyalkyl such as benzoyloxymethyl, benzoyloxyethyl; arylalkylcarbonyloxyalkyl such as benzylcarbonyl-oxymethyl, 2-benzylcarbonyloxyethyl; alkoxycarbonylalkyl or cycloalkytoxycarbonyl-alkyl such as methoxycarbonylmethyl, cyclohexyloxycarbonylmethyl, 1-methoxy-carbonyl-1-ethyl; alkoxycarbonyloxyalkyl or cycloalkyloxycarbonyloxyalkyl such as methoxycarbonyloxymethyl, t-butyloxycarbonyloxymethyl, 1-ethoxycarbonyloxy-1-ethyl, 1-cyclohexyloxycarbonyloxy-1-ethyl; aryloxycarbonyloxyalkyl such as 2-(phenoxycarbonyloxy)ethyl, 2-(5-indanyloxycarbonyloxy)-ethyl; alkoxyalkylcarbonyl-oxyalkyl such as 2-(1-methoxy-2-methylpropan-2-oyloxy)-ethyl; arylalkyloxycarbonyl-oxyalkyl such as 2-(benzyloxycarbonyloxy)ethyl; arylalkenyloxycarbonyloxyalkyl such as 2-(3-phenylpropen-2-yloxycarbonyloxy)ethyl; alkoxycarbonylaminoalkyl such as t-butyloxycarbonylaminomethyl; alkylaminocarbonylaminoalkyl such as methylamino-carbonylaminomethyl; alkanoylaminoalkyl such as acetylaminomethyl; heterocyclic-carbonyloxyalkyl such as 4-methylpiperazinylcarbonyloxymethyl; dialkylamino-carbonylalkyl such as dimethylaminocarbonylmethyl, diethylaminocarbonylmethyl; (5-(loweralkyl)-2-oxo-1,3-dioxolen-4-yl)alkyl such as (5-t-buryl-2-oxo-1,3-dioxolen-4-yl)methyl; and (5-phenyl-2-oxo-1,3-dioxolen-4-yl)alkyl such as (5-phenyl-2-oxo-1,3-dioxolen-4-yl)methyl. Representative amide carboxy protecting groups comprise, without limitation, aminocarbonyl and loweralkylaminocarbonyl groups. Of the above carboxy-protecting groups, loweralkyl, cycloalkyl or arylalkyl ester, for example, methyl ester, ethyl ester, propyl ester, isopropyl ester, butyl ester, sec-butyl ester, isobutyl ester, amyl ester, isoamyl ester, octyl ester, cyclohexyl ester, phenylethyl ester and the like or an alkanoyloxyalkyl, cycloalkanoyloxyalkyl, aroyloxyalkyl or an arylalkylcarbonyloxyalkyl ester are preferred. Preferred amide carboxy protecting groups are loweralkylamino-carbonyl groups.

In the solid phase peptide synthesis method, the α-C-terminal amino acid is attached to a suitable solid support or resin. Suitable solid supports useful for the above synthesis are those materials that are inert to the reagents and reaction conditions of the stepwise condensation-deprotection reactions, as well as being insoluble in the media used. The preferred solid support for synthesis of α-C-terminal carboxy peptides is 4-hydroxymethylphenoxyacetyl-4'-methylbenzyhydrylamine resin (HMP resin). The preferred solid support for α-C-terminal amide peptides is an Fmoc-protected Ramage resin, manufactured and sold by Bachem Inc., California.

At the end of the solid phase synthesis, the peptide is removed from the resin and deprotected, either in successive operations or in a single operation. Removal of the peptide and deprotection can be accomplished conventionally in a single operation by treating the resin-bound polypeptide with a cleavage reagent comprising thioanisole, triisopropyl silane, phenol, and trifluoroacetic acid. In cases wherein the α-C-terminal of the peptide is an alkylamide, the resin is cleaved by aminolysis with an alkylamine. Alternatively, the peptide may be removed by transesterification, e.g. with methanol, followed by aminolysis or by direct transamidation. The protected peptide may be purified at this point or taken to the next step directly. The removal of the side chain protecting groups is accomplished using the cleavage mixture described above. The fully deprotected peptide can be purified by a sequence of chromatographic steps employing any or all of the following types: ion exchange on a weakly basic resin (acetate form); hydrophobic adsorption chromatography on underivatized polystyrene-divinylbenzene (such as Amberlite XAD^{™}); silica gel adsorption chromatography; ion exchange chromatography on carboxymethylcellulose; partition chromatography, e.g. on Sephadex G-25^{™}, LH-20^{™} or countercurrent distribution; high performance liquid chromatography (HPLC), especially reverse-phase HPLC on octyl- or phenyl/hexylsilyl-silica bonded phase column packing. Anyone of ordinary skill in the art will be able to determine easily what would be the preferred chromatographic steps or sequences required to obtain acceptable purification of the C34 peptide.

Molecular weights of these peptides are determined using Electrospray mass spectroscopy.

The present C34 derivatives may be used alone or in combination to optimize their therapeutic effects. They can be administered in a physiologically acceptable medium, e.g. deionized water, phosphate buffered saline (PBS), saline, aqueous ethanol or other alcohol, plasma, proteinaceous solutions, mannitol, aqueous glucose, alcohol, vegetable oil, or the like. Other additives which may be included include buffers, where the media are generally buffered at a pH in the range of about 5 to 10, where the buffer will generally range in concentration from about 50 to 250 mM, salt, where the concentration of salt will generally range from about 5 to 500 mM, physiologically acceptable stabilizers, and the like. The compositions may be lyophilized for convenient storage and transport.

The C34 derivatives may be administered parenterally, such as intravascularly (IV), intraarterially (IA), intramuscularly (IM), subcutaneously (SC), or the like. Administration may in appropriate situations be by transfusion. In some instances, where reaction of the functional group is relatively slow, administration may be oral, nasal, rectal, transdermal or aerosol, where the nature of the conjugate allows for transfer to the vascular system. Usually a single injection will be employed although more than one injection may be used, if desired. The peptide derivative may be administered by any convenient means, including syringe, trocar, catheter, or the like. The particular manner of administration will vary depending upon the amount to be administered, whether a single bolus or continuous administration, or the like. Preferably, the administration will be intravascularly, where the site of introduction is not critical to this invention, preferably at a site where there is rapid blood flow, e.g., intravenously, peripheral or central vein. Other routes may find use where the administration is coupled with slow release techniques or a protective matrix. The intent is that the C34 derivative be effectively distributed in the blood, so as to be able to react with the blood components. The concentration of the conjugate will vary widely, generally ranging from about 1 pg/ml to 50 mg/ml. The total administered intravascularly will generally be in the range of about 0.1 mg/ml to about 10 mg/ml, more usually about 1 mg/ml to about 5 mg/ml.

By bonding to long-lived components of the blood, such as immunoglobulin, serum albumin, red blood cells and platelets, a number of advantages ensue. The activity of the C34 derivatives is extended for days to weeks. Only one administration need to be given during this period of time. Greater specificity can be achieved, since the active compound will be primarily bound to large molecules, where it is less likely to be taken up intracellularly to interfere with other physiological processes.

The formation of the covalent bond between the blood component may occur *in vivo* or *ex vivo.* For *ex vivo* covalent bond formation, the C34 derivative is added to blood serum or a saline solution containing purified blood components such as human serum albumin or IgG, to permit covalent bond formation between the derivative and the blood component. In a preferred format, the C34 derivative is reacted with human serum albumin in saline solution. After formation of the conjugate, the latter may be administered to the subject or lyophilized.

The blood of the mammalian host may be monitored for the activity of the C34 peptide and/or presence of the C34 derivatives. By taking a blood sample from the host at different times, one may determine whether C34 peptide has become bonded to the long-lived blood components in sufficient amount to be therapeutically active and, thereafter, the level of C34 in the blood. If desired, one may also determine to which of the blood components C34 is covalently bonded. Monitoring may also take place by using assays of C34 activity, HPLC-MS or antibodies directed to C34.

The following examples are provided to illustrate preferred embodiments of the invention and shall by no means be construed as limiting its scope.

The present C34 derivatives can be administered to patients according to the methods described below and other methods known in the art. Effective therapeutic dosages of the present C34 derivatives may be determined through procedures well known by those in the art and will take into consideration any concerns over potential toxicity of C34.

The present C34 derivative can also be administered prophylactically to previously uninfected individuals. This can be advantageous in cases where an individual has been subjected to a high risk of exposure to a virus, as can occur when individual has been in contact with an infected individual where there is a high risk of viral transmission. This can be expecially advantageous where there is known cure for the virus, such as the HIV virus. As an example, prophylactic administration of a C34 derivative would be advantageous in a situation where a health care worker has been exposed to blood from an HIV-infected individual, or in other situations where an individual engaged in high-risk activities that potentially expose that individual to the HIV virus.

The invention having been fully described can be further appreciated and understood with reference to the following non-limiting examples.

### GENERAL

Unless stated otherwise, the synthesis of each C34 derivative was performed using an automated solid-phase procedure on a Symphony Peptide Synthesizer with manual intervention during the generation of the derivative. The synthesis was performed on Fmoc-protected Ramage amide linker resin, using Fmoc-protected amino acids. Coupling was achieved by using O-benzotriazol-1-yl-*N,N,N',N'*-tetramethyl-uronium hexafluorophosphate (HBTU) as activator in *N,N*-dimethylformamide (DMF) solution and diisopropylethylamine (DIEA) as base. The Fmoc protective group was removed using 20% piperidine/DMF. When needed, a Boc-protected amino acid was used at the N-terminus in order to generate the free N_{α}-terminus after the peptide is cleaved from resin. All amino acids used during the synthesis possess the L-stereochemistry. Glass reaction vessels were used during the synthesis.

The present invention will now be further disclosed in the following examples. The examples are for illustrative purpose only and are not intended to be limiting.

### Example 1

### Compound A

**Step 1:** The example describes the solid phase peptide synthesis of the compound on a 100 µmole scale. The following protected amino acids were sequentially added to resin: Fmoc-Leu-OH, Fmoc-Leu-OH, Fmoc-Glu(tBu)-OH, Fmoc-Gln(Trt)-OH, Fmoc-Glu(tBu)-OH, Fmoc-Asn(Trt)-OH, Fmoc-Ser(tBu)-OH, Fmoc-Glu(tBu)-OH, Fmoc-Gln(Trt)-OH, Fmoc-Gln(Trt)-OH, Fmoc-Asn(Trt)-OH, Fmoc-Gln(Trt)-OH, Fmoc-Ser(tBu)-OH, Fmoc-Glu(tBu)-OH, Fmoc-Glu(tBu)-OH, Fmoc-Ile-OH, Fmoc-Leu-OH, Fmoc-Ser(tBu)-OH, Fmoc-Thr(tBu)-OH, Fmoc-Tyr(tBu)-OH, Fmoc-Asn(Trt)-OH, Fmoc-Asn(Trt)-OH, Fmoc-Ile-OH, Fmoc-Glu(tBu)-OH, Fmoc-Arg(Pbf)-OH, Fmoc-Asp(tBu)-OH, Fmoc-Trp(Boc)-OH, Fmoc-Glu(tBu)-OH, Fmoc-Met-OH, Fmoc-Trp(Boc)-OH. They were dissolved in *N,N*-dimethylformamide (DMF) and, according to the sequence, activated using O-benzotriazol-1-yl-*N, N, N', N*'-tetramethyl-uronium hexafluorophosphate (HBTU) and diisopropylethylamine (DIEA). Removal of the Fmoc protecting group was achieved using a solution of 20% (V/V) piperidine in *N,N*-dimethylformamide (DMF) for 20 minutes (step 1). The amino group of the final amino acid was acetylated using acetic acid activated using O-benzotriazol-1-yl-*N*, *N*, *N*', *N*'-tetramethyl-uronium hexafluorophosphate (HBTU) and diisopropylethylamine (DIEA).
**Step 2**: The synthesis was continued for the addition of the 3-maleimidopropionic acid (Step 2). Between every coupling, the resin was washed 3 times with *N,N*-dimethylformamide (DMF) and 3 times with isopropanol (ⁱPrOH).
Step 3: The peptide was cleaved from the resin using 85% TFA/5% TIS/5% thioanisole and 5% phenol, followed by precipitation by dry-ice cold Et₂O (Step 3).

### Example 2

### Compound B

**Step 1**: The example describes the solid phase peptide synthesis of the compound on a 100 µmole scale. The following protected amino acids were sequentially added to resin: Fmoc-Leu-OH, Fmoc-Leu-OH, Fmoc-Glu(tBu)-OH, Fmoc-Gln(Trt)-OH, Fmoc-Glu(tBu)-OH, Fmoc-Asn(Trt)-OH, Fmoc-Ser(tBu)-OH, Fmoc-Glu(tBu)-OH, Fmoc-Gln(Trt)-OH, Fmoc-Gln(Trt)-OH, Fmoc-Asn(Trt)-OH, Fmoc-Gln(Trt)-OH, Fmoc-Ser(tBu)-OH, Fmoc-Glu(tBu)-OH, Fmoc-Glu(tBu)-OH, Fmoc-Ile-OH, Fmoc-Leu-OH, Fmoc-Ser(tBu)-OH, Fmoc-Thr(tBu)-OH, Fmoc-Tyr(tBu)-OH, Fmoc-Asn(Trt)-OH, Fmoc-Asn(Trt)-OH, Fmoc-Ile-OH, Fmoc-Glu(tBu)-OH, Fmoc-Arg(Pbf)-OH, Fmoc-Asp(tBu)-OH, Fmoc-Trp(Boc)-OH, Fmoc-Glu(tBu)-OH, Fmoc-Met-OH, Fmoc-Trp(Boc)-OH. They were dissolved in *N,N-*dimethylformamide (DMF) and, according to the sequence, activated using O-benzotriazol-1-yl-*N, N, N', N'-*tetramethyl-uronium hexafluorophosphate (HBTU) and diisopropylethylamine (DIEA). Removal of the Fmoc protecting group was achieved using a solution of 20% {V/V) piperidine in *N*,*N*-dimethylformamide (DMF) for 20 minutes (step 1). The amino group of the final amino acid was acetylated using acetic acid activated using O-benzotriazol-1-yl-*N, N, N'*, *N'*-tetramethyl-uronium hexafluorophosphate (HBTU) and diisopropylethylamine (DIEA).
**Step 2:** The synthesis was continued for the addition of the FMOC-AEEA-OH and the 3-maleimidopropionic acid (Step 2). Between every coupling, the resin was washed 3 times with *N*,*N*-dimethylformamide (DMF) and 3 times with isopropanol (ⁱPrOH).
**Step 3:** The peptide was cleaved from the resin using 85% TFA/5% TIS/5% thioanisole and 5% phenol, followed by precipitation by dry-ice cold Et₂O(Step 3).

### Cellular anti-HIV assay (MTT assay)

The antiviral activity was determined as described in *Journal of Virological Methods,* 1988, 20, 309-321. Briefly, various concentrations of the test compound were brought into each well of a flat-bottom microtiter plate. Subsequently, HIV strain (HIV-1 IIIB) and MT-4 cells were added to a final concentration of 200 CCID₅₀/well and 30,000 cells/well, respectively. In order to determine the toxicity of the test compound, mock-infected cell cultures containing an identical compound concentration range, were incubated in parallel with the HIV-infected cell cultures. After 5 days of incubation (37°C, 5% CO₂), the viability of the cells was determined by the tetrazolium colorimetric MTT method. The results of both assays appear in Table 2 below.

**Table 2**

| Compound | Comment | Antiviral assay IC50 (µM) |
|---|---|---|
| A | quenched | 0.3171 |
| | HSA conjugate | 0.6602 |
| B | quenched | 0.0015 |
| | HSA conjugate | 0.0175 |

## Claims

1. A compound of Formulae I-II

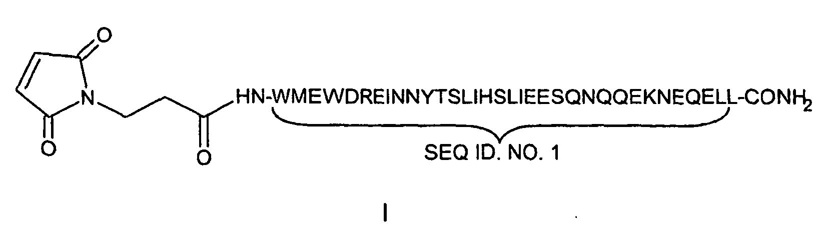

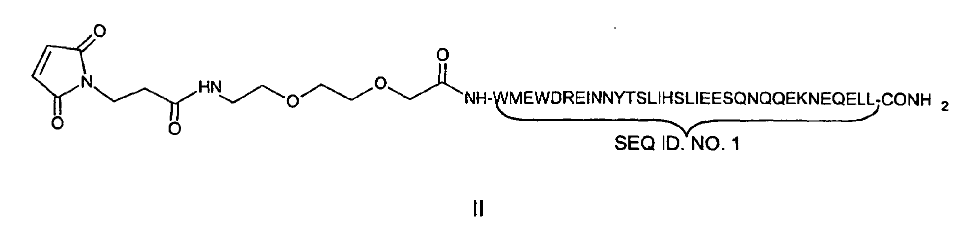

2. A pharmaceutical composition comprising a compound according to claim 1 in combination with a pharmaceutically acceptable carrier.

3. A composition according to claim 2 for inhibiting the activity of HIV, RSV, HPV, MeV or SIV.

4. The use of a compound according to claim 1, alone or in combination with a pharmaceutically acceptable carrier, for the manufacture of a medicament for the inhibition of the activity of HIV, RSV, HPV, MeV or SIV.

5. The use of a compound according to claim 4, for the manufacture of a medicament for the inhibition of the activity of HIV, RSV, HPV, MeV or SIV, said compound being covalently bonded to a blood component.

6. The use of a compound according to claim 4, for the manufacture of a medicament for the inhibition of the activity of HIV, RSV, HPV, MeV or SIV, said compound being capable of covalently bonding to a blood component *in vivo.*

7. A conjugate comprising a compound according to claim 1, covalently bonded to a blood component.

## Patentansprüche

1. Verbindung der Formeln I-II

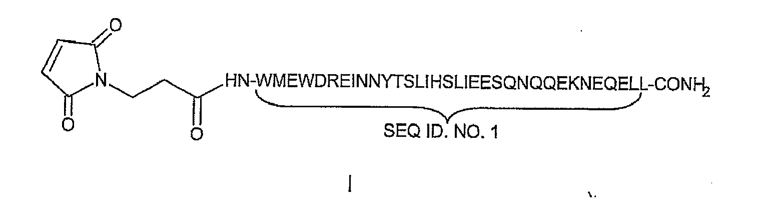

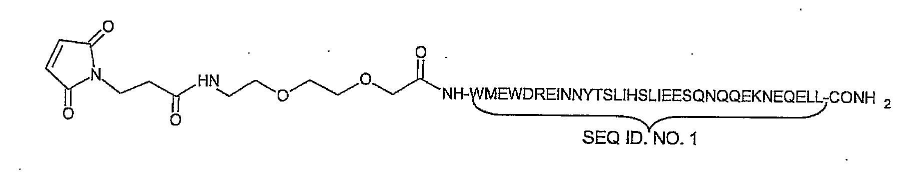

2. Pharmazeutische Zusammensetzung, umfassend eine Verbindung nach Anspruch 1 in Verbindung mit einem pharmazeutisch annehmbaren Träger.

3. Zusammensetzung nach Anspruch 2 zur Hemmung der Aktivität von HIV, RSV, HPV, MeV oder SIV.

4. Verwendung einer Verbindung nach Anspruch 1, allein oder in Verbindung mit einem pharmazeutisch annehmbaren Träger, für die Herstellung eines Medikaments zur Hemmung der Aktivität von HIV, RSV, HPV, MeV oder SIV.

5. Verwendung einer Verbindung nach Anspruch 4 für die Herstellung eines Medikaments zur Hemmung der Aktivität von HIV, RSV, HPV, MeV oder SIV, wobei diese Verbindung kovalent an einen Blutbestandteil gebunden ist.

6. Verwendung einer Verbindung nach Anspruch 4 für die Herstellung eines Medikaments zur Hemmung der Aktivität von HIV, RSV, HPV, MeV oder SIV, wobei diese Verbindung dazu fähig ist, kovalent an einen Blutbestandteil *in vivo* zu binden.

7. Konjugat, umfassend eine Verbindung nach Anspruch 1, die kovalent an einen Blutbestandteil gebunden ist.

## Revendications

1. Un composé correspondant à la formule I ou II

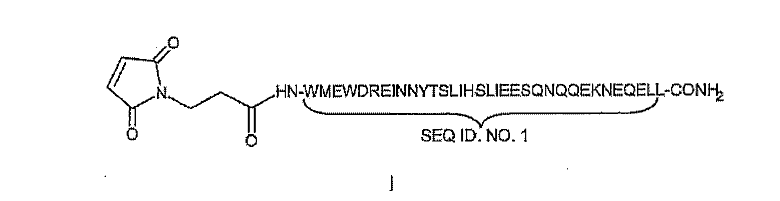

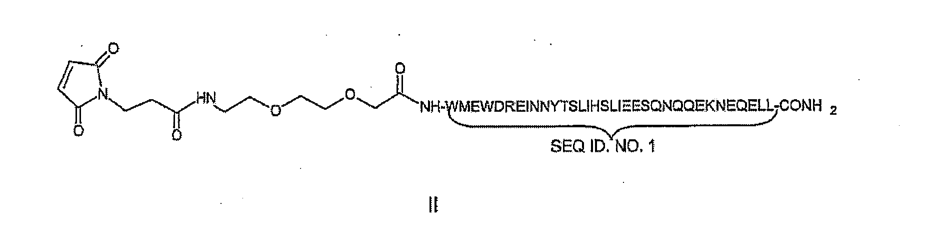

2. Une composition pharmaceutique comprenant un composé selon la revendication 1 en combinaison avec un véhicule pharmaceutiquement acceptable.

3. Une composition selon la revendication 2 destiné à inhiber l'activité de HIV, RSV, HPV, MeV ou SIV.

4. L'usage d'un composé selon la revendication 1, seul ou en combinaison avec un véhicule pharmaceutiquement acceptable, en vue de la fabrication d'un médicament destiné à inhiber l'activité de HIV, RSV, HPV, MeV ou SIV.

5. L'usage d'un composé selon la revendication 4, en vue de la fabrication d'un médicament destiné à inhiber l'activité de HIV, RSV, HPV, MeV ou SIV, ledit composé a été lié par liaison covalente à un composant sanguin.

6. L'usage d'un composé selon la revendication 4, en vue de la fabrication d'un médicament destiné à inhiber l'activité de HIV, RSV, HPV, MeV ou SIV, ledit composé a été lié par liaison covalente à un composant sanguin in vivo.

7. Un conjugué comprenant un composé selon la revendication 1, lié par liaison covalente à un composant sanguin.


### SEQUENCE LISTING

<110> ConjuChem Inc.
<120> LONG LASTING FUSION PEPTIDE INHIBITORS FOR HIV INFECTION
<130> 53124
<140> EP 04015696.0
<141> 2004-07-03
<160> 1
<170> PatentIn version 3.1
<210> 1
<211> 34
<212> PRT
<213> Artificial
<221 > PEPTIDE
<222> (1)..(34)
<400> 1
   Trp Met Glu Trp Asp Arg Glu Ile Asn Asn Tyr Thr Ser Leu Ile His
   1 5 10 15
   Ser Leu Ile Glu Glu Ser Gln Asn Gln Gln Glu Lys Asn Glu Gln Glu
   20 25 30
   Leu Leu